**Europäisches Patentamt**

**European Patent Office**

**Office européen des brevets**

(11) Veröffentlichungsnummer: **0 040 717**
A1

(12) ## EUROPÄISCHE PATENTANMELDUNG

(21) Anmeldenummer: **81103380.2**

(22) Anmeldetag: **05.05.81**

(51) Int. Cl.³: **A 61 F 5/01,** A 61 F 1/04

(30) Priorität: **27.05.80 DE 3020083**

(43) Veröffentlichungstag der Anmeldung: **02.12.81**
**Patentblatt 81/48**

(84) Benannte Vertragsstaaten: **AT CH FR GB IT LI NL SE**

(71) Anmelder: **Will, Peter, Dr., Lerchenweg 5,
D-6109 Mühltal 1 (DE)**

(72) Erfinder: **Will, Peter, Dr., Lerchenweg 5,
D-6109 Mühltal 1 (DE)**

(54) **Gelenkführungsschiene.**

(57) Vorrichtung zur Unterstützung von Gelenken für orthopädische und chirurgische Zwecke, die eine Anwendung in der Hauptbewegungsebene auf der Gelenkperipherie bzw. Oberfläche gestattet. Die Mitbewegung und Anpassung der Schiene an die Formveränderung der Gelenkoberfläche bei Bewegungen wird durch eine Mehrgliedrigkeit der Gelenkführungsschiene gewährleistet, wobei die Glieder (1a, 1b, 1c), die als stumpfwinklige Winkelhebel ausgebildet sind, derart durch Gelenke miteinander zu einer Kette vereinigt sind, dass sie bei einer radialen Bewegung der Schienenglieder (1a, 1b, 1c) zugleich eine Streckung der Schiene nach dem Prinzip der Nürnberger Schere bewirken.

0040717

Dr. med. Peter Will, Lerchenweg 5, 6109 Mühltal (Hessen)


Gelenkführungsschiene


Bei der Erkrankung der Körpergelenke werden häufig Maßnahmen erforderlich, die den Bandapparat der Gelenke stabilisieren und die gelenkbewegenden Organe unterstützen sollen. Hierzu finden zirkuläre, den gesamten Gelenkbereich umfassende, dehnbare Bandagen und Korsettagen für den Rumpf oder Schienenapparate mit eingerichteten Gelenken Anwendung. Die Bauweise der organischen Gelenke und ihrer Weichteilumkleidung gibt allerdings bei der Versorgung mit mechanischen, gelenkstabilisierenden Maßnahmen Probleme auf, die bisher nur unzulänglich gelöst sind. Im Gegensatz zu den starren, flächenschlüssigen Kunstgelenken der Technik, sind Körpergelenke infolge ihrer unregelmäßigen Form der artikulierenden Gelenkflächen durch eine inkonstante Lage der Gelenkachse charakterisiert. Einachsige Gelenke, wie sie in Schienenapparaten verwendet werden, sind deshalb auf Körpergelenke nicht zu zentrieren. Schuberscheinungen des Apparates bei Gelenkbewegungen und scherende Kräfte im Gelenkbereich sind bekannte, nachteilige Erscheinungen. Auch die Versorgung erkrankter Gelenke mit Bandagen und Orthesen ist oft mit Schwierigkeiten verbunden, da bei Bewegungsvorgängen erhebliche Verschiebungen an den oberflächlichen, gelenkumhüllenden Weichteilen auftreten. Die dazu verwendeten elastischen, dehnbaren Materialien als Bandagen sind nur bedingt geeignet, sich den ständig wechselnden Weichteilveränderungen anzupassen. Mit der Verwendung festerer, starrer Materialien zur Erzeugung größerer Stabilität sind oft Druck- und Stauchungserscheinungen durch die therapeutischen Vorrichtungen mit hierdurch bedingten nachteiligen Einflüssen auf die Weichteile, Nerven- und Blutgefäßbahnen der behandelten Extremität verbunden.

Die genannten Nachteile bei der Behandlung von Gelenkerkrankungen werden durch die erfindungsgemäße Schienenkonstruktion vermieden. Nach der Erfindung handelt es sich um eine Schiene zur Unterstützung von Gelenken für orthopädische oder chirurgische Zwecke, dadurch gekennzeichnet, daß sie aus einer Kette von stumpfwinkeligen Winkelhebeln besteht, bei der der vorzugsweise längere Schenkel jedes nachfolgenden Winkelhebels sich mit dem Ende des entsprechenden Schenkels des vorhergehenden Winkelhebels überlappt und der Gelenkpunkt jedes Winkelhebels mit Ausnahme des ersten in einem Längsschlitz am Ende des vorzugsweise längeren Schenkels des vorhergehenden Winkelhebels geführt ist und jeder Gelenkpunkt mit Ausnahme des letzten zusätzlich mittels einer Lasche mit dem Ende des vorzugsweise kürzeren und nicht mit dem Längsschlitz versehenen Schenkels des nachfolgenden Winkelhebels gelenkig so verbunden ist, daß bei einer Biegung der gesamten Kette diese sich nach dem Prinzip der Nürnberger Schere gleichzeitig streckt.

Im Gegensatz zu den üblicherweise angewendeten Gelenken in den Behandlungsschienen, die möglichst auf die Achse der Körpergelenke zu zentrieren sind, wird die neue Schiene in der Hauptbewegungsebene eines Gelenkes auf der Gelenkperipherie bzw. Oberfläche eingerichtet und an den Gelenkarmen bzw. Extremitäten befestigt. Die Mitbewegung und Anpassung der Schiene an die Formveränderung der Gelenkoberfläche bei Bewegung wird durch die Mehrgliedrigkeit der Schiene mit Gelenkverbindungen, die eine Längenveränderung der Schiene in Abhängigkeit zu einer radialen Bewegung der einzelnen Glieder zulassen, erreicht.

Die Maße der einzelnen Glieder und der Winkel, in dem die Schienenglieder ausgeführt sind, lassen sich den speziellen Forderungen anpassen. Dies trifft beispielsweise auf Ausführungen für Erwachsene und Kinder oder unterschiedliche Belastungen der Schiene am Rumpf oder Extremitätenbereich zu, wo aus statischen oder Gründen des Umfanges auch die Maße der Schiene bzw. ihrer einzelnen Glieder anzupassen sind.

Ebenso ist auch die schlitzförmige Öffnung nach der Erfindung variabel, da mit ihr die Beweglichkeit in Längsrichtung der Schie-

- 3 -

ne evtl. auch in einer bestimmten Richtung durch Abbiegen des Schlitzes vorgegeben werden kann.

Die Länge der Schiene kann durch die Anzahl der zusammengesteckten Glieder beliebig eingestellt werden. Um stellenweise oder über die ganze Schienenlänge eine Verbreiterung oder Verstärkung zu erhalten, lassen sich mehrere parallel laufende Gliedreihen auf entsprechend verlängerte Achsen aufsetzen, so daß mehrere nebeneinander laufende Kettengliedreihen entstehen.

Bevorzugt werden zur Herstellung der Gelenkführungsschiene serienmäßig vorgefertigte Gliedteile verwendet, die z.B. aus Metallen wie Stahl, Aluminium aber auch aus Kunststoffen wie z.B. Polyäthylen, Polypropylen, Polyamid, Polymethacrylat, Polystrol oder aus armierten z.B. glasfaserverstärkten Produkten bestehen können.

Von Vorteil ist eine Kombination von Metall- und Kunststoffgliedern um die mechanische Belastbarkeit zu verbessern ohne das Gewicht wesentlich zu erhöhen. z.B. lassen sich dünne Metallglieder (1a, 1b, 2a) - Dicke o,1 bis etwa 5 mm zwischen stärkere Kunststoffglieder (Dicke 3 - 50 mm) einschieben.

Man kann die Seitenteile der Kunststoffglieder mit Stahleinlagen ausstatten und hohle oder skelettierte Metallglieder verwenden. Zwischen Metallgliedern können Abstandhalter aus Kunststoff eingeschaltet werden, die die Fläche der Schienen in der Breite vergrößern. Werden weiche Materialien wie Kautschuk oder Weich-PVC verwendet, so kann man der Schiene elastische Eigenschaften verleihen und dies mit Einsetzen entsprechender Einzelglieder auf bestimmte Bereiche beschränken.

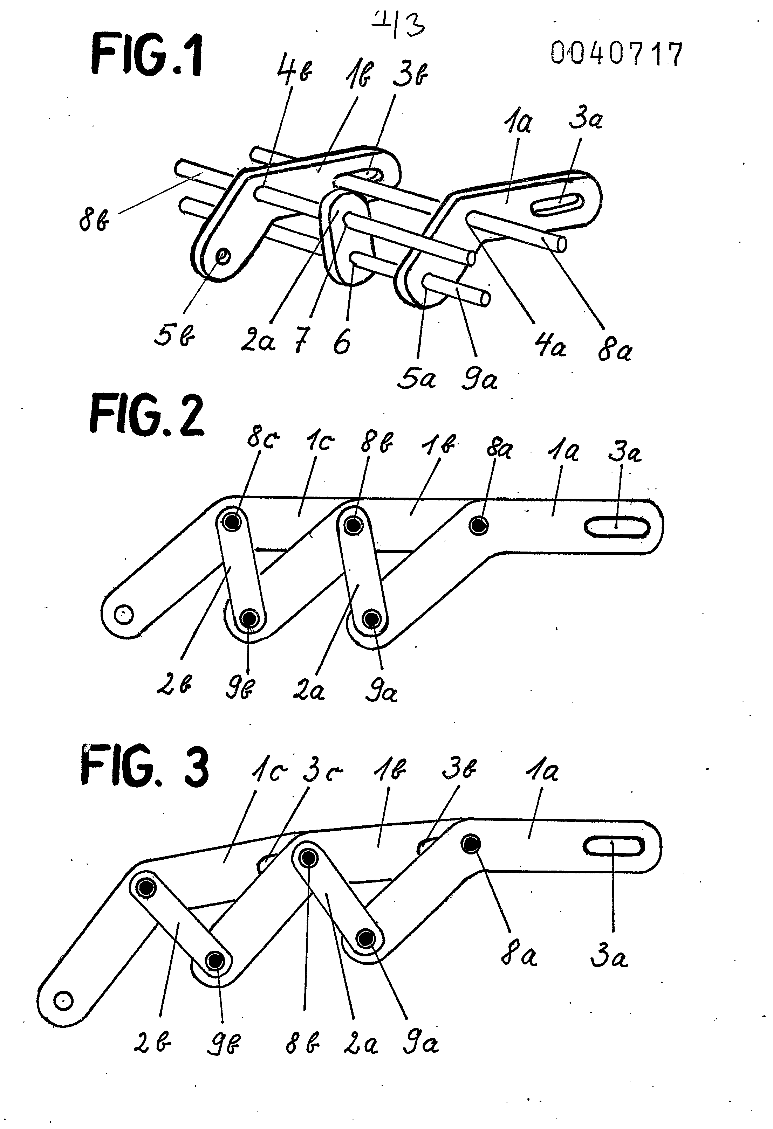

In Fig. 1 werden zwei Schienenglieder 1a und 1b dargestellt sowie ein Verbindungsglied 2a. Jedes Schienenglied 1a u. 1b ist winkelförmig gestaltet und mit einem Führungsschlitz 3a und 3b sowie mit zwei Bohrungen 4a und 4b sowie 5a und 5b versehen. Die Bohrungen 4a und 5a nehmen zwei Gestänge als Achsen 8a und 9a auf. Das Schienenglied 1b ist mit seinem Führungsschlitz 3b auf die Achse 8a aufgeschoben. Das Verbindungsglied 2a, welches mit seinen Bohrlöchern 6 und 7 auf die parallel gelagerten Achsen 8a und 9a aufgeschoben ist, stellt eine Verbindung zwischen den Schienengliedern 1a und 1b her.

Fig. 2 zeigt die Anordnung von drei Schienengliedern 1a, 1b und 1c in einer Ausgangsposition.

Fig. 3 demonstriert einen möglichen Bewegungsablauf des Erfindungsgegenstandes. Hieraus wird ersichtlich, daß die einzelnen Schienenglieder 1a, 1b und 1c nunmehr in einem Bogen aneinander gereiht sind und daß sich der erfolgte Bewegungsablauf aus zwei Komponenten zusammensetzt. Die Schienenglieder 1b und 1c sind jeweils in einem Winkel radialwärts um die Achse 9a bzw. 9b weiterbewegt worden, wobei die Verbindungsglieder 2a und 2b zur Lenkung des Bewegungsablaufes dienen. Darüberhinaus wird ersichtlich, daß jedes Schienenglied 1b und 1c aus seiner Ausgangsposition in Längsrichtung der Schiene als zweite Bewegungskomponente verschoben ist, wobei dieser Bewegungsvorgang durch die Führungsschlitze 3b und 3c mit den Achsen 8a und 8b gelenkt wird.

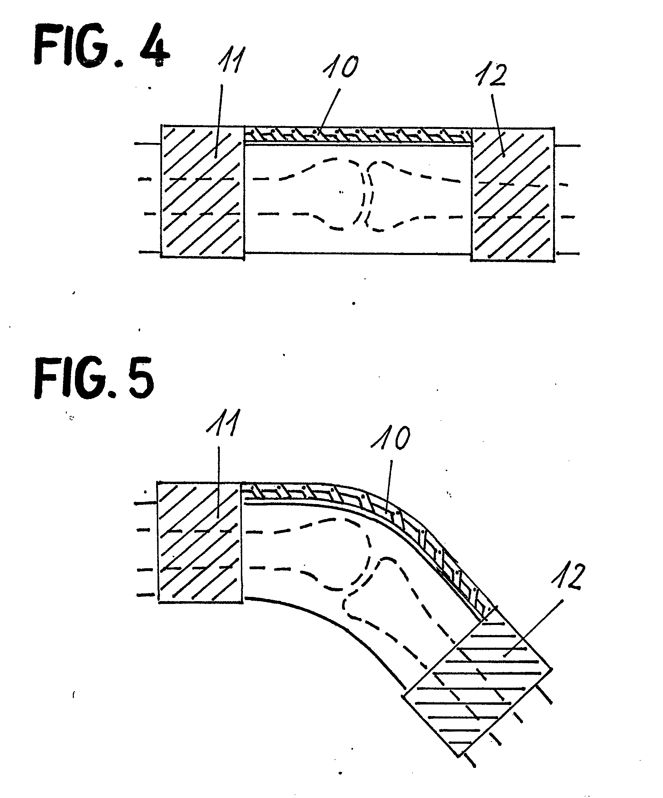

Fig. 4 und 5 zeigen ein Anwendungsbeispiel. Die mehrgliedrige Führungsschiene 10 ist mit Schellen 11 und 12 an den Gelenkarmen bzw. Extremitäten proximal- und distalwärts des Gelenkes befestigt und über der Gelenkstreckseite lokalisiert. Bei einer Beugebewegung des Gelenkes können die Schienenglieder durch den ihnen gegebenen Bewegungsspielraum der Gelenkoberfläche folgen und sich der entsprechenden Formveränderung anpassen. Bei der Streckung des Gelenkes schieben sich die Schienenglieder in ihre Ausgangsposition zurück.

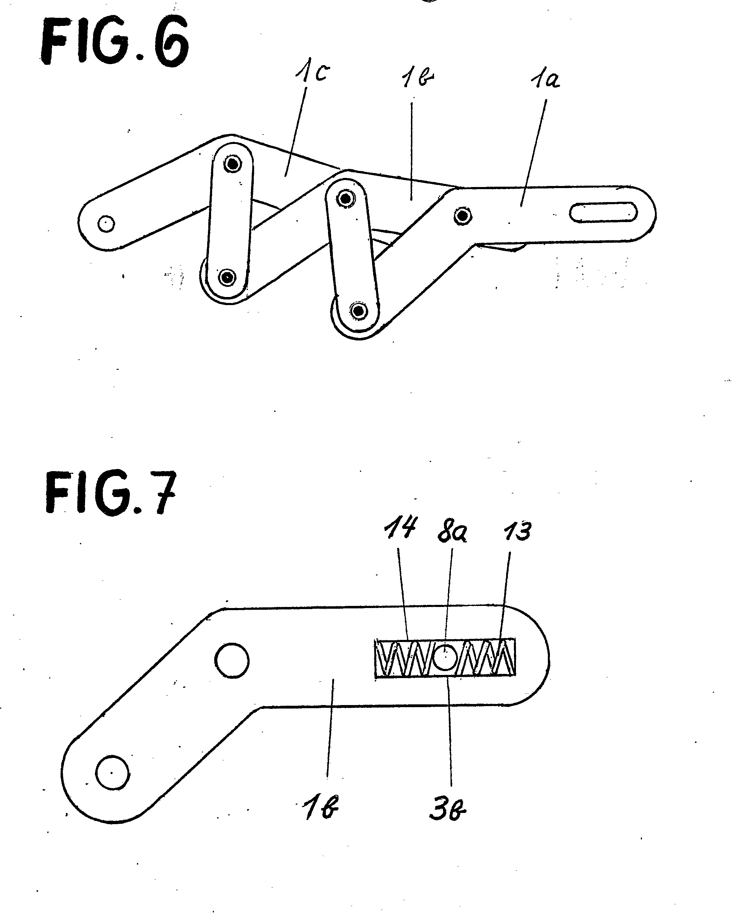

Fig. 6 zeigt einen weiteren möglichen Bewegungsablauf in der Führungsschiene. Durch eine Verlängerung der Führungsschlitze kann ein zur Fig. 3 gegenläufiger Bewegungsvorgang der Schienenglieder 1a, 1b und 1c ermöglicht werden.

Fig. 7 zeigt eine weitere Ausgestaltung der Schiene, indem in einem Führungsschlitz 3b eines Gliedes 1b zwei federnde Elemente 13 und 14 eingesetzt sind, so daß die Bewegung des Gliedes 1b gegenüber der Achse 8a nach den Erfordernissen erschwert oder aber auch erleichtert werden kann. Die federnden Elemente können wahlweise auch nur für eine Seite eingesetzt sein, wobei der Bewegungsvorgang der Schiene nur in einer gewünschten Richtung beeinflußt wird.

Die Anordnung der Verbindungs- und Schienenglieder auf parallel gerichteten Achsen gestattet lediglich Bewegungsabläufe und Formveränderungen der Schiene in einer Ebenen parallel zu ihrer Längsachse. Knickbildungen oder kurzwinklige Abbiegungen sind im Verlauf der mehrgliedrigen Schiene ausgeschlossen. Die Aufgabe der Gelenkführung und Stabilisierung einer Funktionsrichtung wird durch die konstruktiven Eigenheiten der mehrgliedrigen Schiene in besonders günstiger Weise gelöst. Durch eine Umhüllung der Schiene mit elastischen, der Formveränderung der Schiene sich anpassenden Materialien werden störende mechanische Irritationen der Weichteile weitgehend verhindert. Je nach Zweckmäßigkeit in der Anwendung läßt sich die Wirkungsweise der mehrgliedrigen Schiene durch konstruktive Änderungen und mechanische Zusätze erweitern und variieren. Soll nicht nur eine Gelenkführung und Stabilisierung erreicht werden sondern auch eine Unterstützung der gelenkbewegenden Organe, so lassen sich zwischen die einzelnen Schienenglieder federnde Elemente einfügen, die entweder eine Rückbewegung der einzelnen Schienenglieder in die Neutralstellung oder aber eine Bewegung in eine vorgegebene Richtung bewerkstelligen. Hierzu eignen sich federnde Elemente oder Kraftspeicher, die jeweils auf zwei Glieder Einfluß nehmen (Fig. 7) oder auch solche, die mehrere Glieder gleichzeitig überbrücken und beispielsweise an den Schienenenden befestigt sind.

Muß die Bewegung eines erkrankten Gelenkes teilweise gesperrt werden, so kann durch Blockierung der einzelnen Schienenglieder der Umfang der Formveränderung der Schiene und somit eine Einschränkung der Gelenkfunktion durch die Führungsschiene erreicht werden, wie z.B. durch das Aufsetzen einer U-förmigen Steckverbindung oder durch Verschraubung gewindeführender Achsen zur Verklemmung der Radial- oder auch Längsbewegung der Schienenglieder 1a oder der Führungsglieder 2a.

Da sich die Funktionsabläufe einzelner Bewegungsorgane in verschiedenen Bewegungsebenen abspielen, können zwischen zwei oder auch mehrere Schienenglieder verbindende Elemente eingebaut werden, die entweder durch Gelenke oder flexible Verbindungen eine Anpassung der Schienenkonstruktion an Bewegungsabläufe gestatten. Um eine stabile Auflage der Schienenkonstruktion über einem Bewegungsorgan zu gewährleisten, kann schließlich an jedem der Schienenglieder eine das Bewegungsorgan zirkulär umfassende Halterung oder Bandage befestigt werden.

## Patentansprüche

1. Schiene zur Unterstützung von Gelenken für orthopädische oder chirurgische Zwecke, dadurch gekennzeichnet, daß sie aus einer Kette von stumpfwinkeligen Winkelhebeln bestehen, bei der der vorzugsweise längere Schenkel jedes nachfolgenden Winkelhebels sich mit dem Ende des entsprechenden Schenkels des vorhergehenden Winkelhebels überlappt und der Gelenkpunkt jedes Winkelhebels mit Ausnahme des ersten in einem Längsschlitz am Ende des vorzugsweise längeren Schenkels des vorhergehenden Winkelhebels geführt ist und jeder Gelenkpunkt mit Ausnahme des letzten zusätzlich mittels einer Lasche mit dem Ende des vorzugsweise kürzeren und nicht mit dem Längsschlitz versehenen Schenkels des nachfolgenden Winkelhebels gelenkig so verbunden ist, daß bei einer Biegung der gesamten Kette diese sich nach dem Prinzip der Nürnberger Schere gleichzeitig streckt.

2. Mehrgliedrige Gelenkführungsschiene nach Anspruch 1 dadurch gekennzeichnet, daß federnde Elemente, die in den Längsschlitzen der einzelnen Winkelhebel eingesetzt sind, die Bewegung der einzelnen Schienenglieder in der einen oder anderen Richtung fördern oder hemmen.

FIG.1

1/3

0040717

FIG.2

FIG. 3

# FIG. 4

11    10    12

# FIG. 5

11    10    12

## FIG.6

1c  1b  1a

## FIG.7

14  8a  13

1b  3b

Europäisches
Patentamt

**EUROPÄISCHER RECHERCHENBERICHT**

0040717

Nummer der Anmeldung

EP 81 10 3380

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 4 144 881 (CHAPPEL) <br><br> * Zusammenfassung; Figuren * <br><br> -- | 1,2 |
| | US - A - 3 902 482 (TAYLOR) <br><br> * Spalte 3, Zeile 59 - Spalte 4, Zeile 14; Figuren * <br><br> -- | 1 |
| A | US - A - 3 580 099 (MOSHER) <br><br> * Abbildungen 1-4; Patentanspruch * <br><br> -- | 1 |
| A | DE - C - 351 488 (PRINGLE) <br><br> * Insgesamt * <br><br> ---- | 1 |

**KLASSIFIKATION DER ANMELDUNG (Int. Cl.)**

A 61 F 5/01
1/04

**RECHERCHIERTE SACHGEBIETE (Int. Cl.³)**

A 61 F

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 20-08-1981 | STEENBAKKER |

EPA form 1503.1   06.78